# EUROPEAN PATENT APPLICATION

(11) **EP 0 849 275 A1**
(43) Date of publication of application: **24.06.1998**
(21) Application number: 96202701.7
(22) Date of filing: 26.09.1996
(51) Int. Cl.: C07K 14/35, A61K 39/04

(54) **Mannosylated peptides**

(71) Applicant: Rijksuniversiteit te Leiden, 2312 AV Leiden (NL)
(72) Inventor: The designation of the inventor has not yet been filed
(74) Representative: Smulders, Theodorus A.H.J., Ir.

(57) **Abstract**

T cells mediate specific immune reactions. They recognize (foreign) protein fragments - peptides - that are bound in the peptide binding groove of MHC molecules. These peptide MHC complexes are generated intracellularly and subsequently transported to the cell surface for recoginition by T cells. A rate limiting step in the MHC restricted presentation of (foreign) peptides derived from extracellular sources is therefore the uptake of such antigens by antigen presenting cells and the delivery to the intracellular compartment(s) where the MHC-peptide complexes are formed. Professional antigen presenting cells like dendritic cells and macrophages take up antigens by macropinocytosis and mannose receptor mediated endocytosis. We have explored the possibility that the mannosylation of otherwise non-mannosylated antigens would lead to enhanced uptake and MHC restricted presentation of (fragments of) such antigens by mannose receptor positive cells. Using mannose receptor positive cultured human dendritic cells as antigen presenting cells we found that the mannosylation of antigenic peptides resulted in a 300 - 10.000 fold enhanced potency to stimulate MHC class II restricted peptide-specific T cell clones compared to non-mannosylated peptides. These results indicate that mannosylation of antigens leads to selective targeting and subsequent superior presentation by dendritic cells, a result which may be applicable in for example vaccine design.

## Description

The present invention relates to the field of immunology, in particular cellular immunology. More specifically it relates to the presentation of antigen-derived peptides on the surface of antigen presenting cells (APC's) in the context of an MHC-molecule.

T cells play a central role in the immune system. They mediate specific immune reactions against pathogens and tumors but also cause autoimmune diseases and are responsible for the initiation of transplant rejection. Research in the last ten years has revealed that T cells recognize protein fragments - peptides - that are bound in the peptide binding groove of MHC-molecules. These peptide MHC-complexes are generated intracellularly and subsequently transported to the cell surface in order to allow detection by T cells. Peptides derived from intracellularly synthetized proteins most commonly are found in association with MHC-class I molecules. Peptides derived from endocytosed proteins synthetized outside the cells are most commonly found in association with MHC-class II molecules. It is well established that primary T cell responses are initiated most efficiently if peptides are presented by MHC-molecules on a specialized class of antigen presenting cells: cells of the dendritic lineage. Dendritic cells are present in low numbers in peripheral blood, in lymphoid tissues and epithelia. Their extraordinary capacity to induce primary immune responses is attributed to the high cell surface expression of MHC-class I and class II molecules, adhesion molecules and high co-stimulatory activity. Other, currently unknown proteins may also contribute to this process.

One of the problems encountered in trying to induce T cell responses to proteinaceous antigens is that peptides are not efficiently presented by APC's. This problem may lie in the uptake of the proteinaceous antigen or a peptide derived therefrom by the APC, in the processing of the antigen (cleavage sites and/or transport signals for transport to the right compartment and eventually the surface of the APC, in the complexing of the peptide with the MHC-molecule, etc). Any of these problems may be the reason why peptide or protein based vaccines for many specific pathogens have met with limited success. The present invention indentifies an important limiting step in antigen processing. Said limiting step is the uptake of proteinaceous antigens or peptides derived therefrom.

The present invention improves the uptake of said proteinaceous antigens or peptides by APC's by providing said antigen or peptide with one or more carbohydrate moieties which has an affinity for a mannose receptor on the surface of an APC.

Recent reports have revealed that both cultured monocyte-derived human dendritic cells and murine dendritic cells in situ express mannose receptors and that this mannose receptor is used for the efficient endocytosis of mannosylated proteins.

Thus the invention provides a method for enhancing uptake of a proteinaceous antigen or a peptide derived therefrom by antigen presenting cells having a mannose or similar receptor, whereby the antigen is provided with at least one and preferably at least two mannose groups or a functional equivalent thereof.

An important aspect of the present invention is that the antigen or peptide is targeted to a mannose receptor present on APC's such as the mannose receptor present on dendritic cells. For this purpose a mannose group (or more than one) attached to the antigen or the peptide is of course very suitable, but other glycosyl groups may also be used as long as they have a significant binding affinity for a mannose receptor. The structure of the total glycosyl group is not overall important, as long as at least one carbohydrate moiety is recognized by the mannose receptor. The number of glycosylic residues may thus also vary. Our experiments show that the number of carbohydrates attached to the peptide (n=1-6 or preferably 2-6) does influence only marginally the presentation of the peptide on the cell surface. It is clear that the use of a different number of carbohydrate moieties on the peptide (n>6) than the number we used will show a comparable effect. Other moieties that have binding affinity for mannose receptors on APC's include, but are not limited to β-L-Fucose, β-D-Glucopyranose, β-D-N-Acetylgalactosamine, etc. This holds true also for modified carbohydrates which have affinity for the mannose receptor like carbohydrate analogs possessing instead of an oxygen atom, an anomeric carbon or an anomeric sulfur atom. It could be a derivative containing one or more halogen atoms replacing one or more hydroxyl(s). It may be modified carbohydrate derivatives with seven, eighth or more atom rings.

The improvement in the uptake by targeting a mannose receptor and the transport by said mannose receptor to the right compartment in the cell for processing, and/or complexing to an MHC-I or MHC-II molecule results in a very efficient presentation of the mannosylated peptide on the surface of the APC's, in particular those of dendritic origin.

Our results demonstrate that mannosylation of peptides leads to very efficient uptake and presentation of these peptides by dendritic cells at low concentrations of peptide. Depending on the T cell clone used, 300 - 10,000 fold less mannosylated peptide is required for the induction of T cell proliferation when compared with non-mannosylated peptides. Furthermore, only a short exposure to mannosylated peptide is required for the generation of sufficient MHC-peptide complexes to allow the induction of T cell proliferation. This indicates that the mannose receptor expressed on the cell surface of cultured dendritic cells mediates very efficient transport of mannosylated peptides to the intracellular compartment were (newly synthetized) MHC-class II molecules bind peptides. We also observed that mannosylated peptides are presented more efficiently than non-mannosylated peptides when human peripheral blood mononuclear cells are used as antigen presenting cells. This shows that enough mannose receptor positive antigen presenting cells are present in these peripheral blood preparations to allow enhanced presentation. It is known that in peripheral blood approximately 1% of the cells are immature dendritic cells that could mediate this effect.

In the peptides we used, the carbohydrate moieties were attached to the N-terminus of the peptides. It is obvious that the same biological effect would be obtained with peptides in which the carbohydrate moieties are attached to the C-terminus, or to parts of the peptides between the N-terminus and the C-terminus e.g. to side chains. Also peptides having carbohydrate moieties attached to combinations of the possibilities mentioned above are expected to have the same biological effect.

It is clear that peptide-like compounds (peptoids and/or peptidomimetics) to which carbohydrates are attached can have the same effect. Peptoids could be peptide-like compounds having a modified backbone structure because they contain unnatural amino acids, D-amino acids, amino acid like building blocks such as β....Ω -amino acids, amide bond mimics like reduced peptide bonds, sulfonamide bonds, sulfinamide bonds, ester bonds, ether bonds, disulfide bonds, thioether bonds or saturated an unsaturated carbon-carbon bonds. Peptoids could be PNA also.

It is unlikely that the way the glycosylic groups are attached to the peptides is of crucial importance for the biological effect. Other ways of attaching the glycosylic group(s) could be the reaction of other amine reactive groups in the glycosylic moiety like active esters, acid chlorides, acid bromides, acid iodides, symmetric anhydrides, unsymmetric anhydrides, in situ preactivated carboxylic acids to amines in the peptides. It could be the reaction of activated carboxylic groups in the peptide to an amine in the glycosylic moiety. It could be the reaction of a thiogroup in the peptide to a thioreactive group in the carbohydrate moiety like an S-Npys, an S-Nps, a thiol, an acetylbromide, an acetyliodide. It could be the reaction between a thiogroup in the carbohydrate to a thiolreactive group in the peptide, like an acetylbromide, an acetyliodide. It could be an ester forming reaction between a activated carboxylic acid, an acid chloride, an acid bromide, an acid iodide, an in situ activated carboxylic acid in the carbohydrate moiety and a hydroxy group in the peptide. It could be any nucleophilic substitution reaction between a modified carbohydrate and a suitable reactive center in the peptide. It could be any addition reaction of a modified carbohydrate and a suitable reactive center in the peptide.

It is evident that this type of compound could be synthesized completely by solid phase strategies. Such compounds could be synthesized by performing peptide synthesis using amino acids bearing carbohydrate moieties in the side chain. These glycopeptides could be made by using amino acids functionalyzed on the α-amino group with carbohydrate moieties that are introduced at the N-terminus of the peptide to a branched cluster of amino acids possessing various amino functionalities.

Multiple glycosylic moieties in the peptide could also be attached to the peptide by a dendrimer type of structure. These compounds can be synthesized by using bis-amino acids (like lysine, ornithine and diaminopropionic acid). During synthesis the protecting groups are removed from both amino groups before coupling of the next amino acid. This procedure yields constructs with a branched structure, with the possibility to couple glycosylic moieties to the branches.

The best results will of course be obtained if the peptides which are provided to the mannose receptor or which are the result of processing of the proteinaceous antigen delivered to the mannose receptor also have the other characteristics favorable to APC presentation in the context of MHC-class I or MHC-class II molecules. These include, the right size to fit on the MHC-molecule, the right anchor residues to fit in the respective MHC-molecules, transport signals for arriving at the MHC-molecule and for the proteinaceous antigen the proper cleavage sites for the processing enzymes of the APC's.

The mannosylated proteins and/or peptides, possibly in combination with mannose receptor expressing dendritic cells or dendritic-like cells, can be used for a number of purposes. These include, but are not limited to:
- The use of mannosylated proteins and/or peptides for vaccination purposes. Amongst others this includes vaccination protocols for the induction of immunity against pathogens like (myco)bacteria, virusses, yeasts, fungi, helminths and parasites but also vaccination protocols designed to enhance or elicit tumor specific responses.
   For instance desired mannosylated (peptide) antigens may comprise (mixtures or combinations of) known antigenic proteins or peptides, or one or more covalently linked known T helper and cytotoxic T cell epitopes. For example, a known T helper cell epitope that evokes a memory response and thus may aid in eliciting a CD8 response to the cytotoxic T cell epitope, for example a tumor specific peptide.
   Another delivery system may utilize mannosylated liposomes or vesicles into which the desired antigenic proteins or peptides have been incorporated.
- T cells reactive with autoantigens have been described and are thought to play a role in the development of autoimmune diseases like type I diabetes and reumathoid arthritis. Similarly, alloreactive T cells, responsible for graft rejection after organ transplantation and the development of graft versus host disease after bone marrow transplantation, have been shown to be often specific for or dependent on the presence of particular MHC bound peptides. Immune intervention can be envisioned at the level of competition for binding to MHC molecules. For this purpose synthetic compounds with high affinity for the peptide binding groove must be designed that are able to compete for binding to MHC molecules and thus block the binding of immunogenic autoantigen derived peptides to these MHC molecules. This approach is hampered by the relative inefficient uptake of peptides by antigen presenting cells and can be facilitated by the use of mannosylated peptides.
- Peptides with antagonistic properties have been described. These peptides differ from the agonist in the nature of the amino acid(s) at T cell receptor contact points and can (partially) block the response to the agonist. The in vivo application of peptides with antagonistic properties may be hampered by the short half-life of peptides in vivo. The use of mannosylated peptides with antagonist properties may be applicable for the efficient targetting of the peptides to dendritic cells and other mannose receptor positive cells and thus escape from degradation.

Vaccines may of course contain usual additives and/or adjuvantia and/or carriers as may other pharmaceutical preparations.

The invention will be explained in more detail in the following experimental part.

### EXPERIMENTAL PART

### Introduction

In our experiments we used various mono- and multiple-mannosylated peptides in which the mannose groups had been attached to the N-terminus of the peptides. For this we synthesized peptides containing in addition an N-terminal lysine and peptides containing in addition various N-terminal lysines (n=1-5).

Mannosylation was performed by reaction of the N-terminal amine group and the amine group(s) in the side chains of the lysine(s) with an isothiocyanate derivative of mannose, yielding an thioureum linkage between the peptide and the mannose moieties.

Herein we use the word mannose frequently. In the experiments the word mannose should be read as mannose, everywhere else the word mannose should be read as "every carbohydrate moiety that shows affinity for the mannose receptor".

The formation and expression of antigenic MHC-peptide complexes was measured using proliferative peptide specific T cell clones. The clones were cultured together with HLA-DR matched cultured dendritic cells in the presence or absence of a concentration range of the appropiate peptide. The peptides were either non-mannosylated or mannosylated. Activation of the T cell clones was measured by the incorporation of ^{[3}H]-thymidine after 48 hours of culture. In these experiments we observed that 300- to 10.000 fold less of the mannosylated peptide was required for the induction of half-maximal proliferation compared to the non-mannosylated peptide.

### MATERIALS AND METHODS

### Synthetic peptides

Peptides were synthesized by solid phase strategies on an automated multiple peptide synthesizer (Abimed AMS 422) (Gausepohl et al., 1990^{a}). TentagelS AC (Rapp, Tübingen, Germany), (Sheppard et al., 1982; Rapp et al., 1990), a graft polymer of polyethyleneglycol and polystyrene to which the C-terminal amino acid of the peptide had been attached by the manufacturer, was used as a resin (loading 0.2 µeq, particle size 90µm). Repetitive couplings were performed by addition of a mixture of 90 µl 0.67 M PyBOP (Gausepohl et al., 1990^{b}; Coste et al., 1990) in NMP, 20 µl NMM in NMP 2/1 (v/v) and 100 µl of a 0.60 M solution of the appropriate Fmoc amino acid (Fields et al., 1990) in NMP (6-fold excess) to each reaction vessel. At 70% of the reaction time approximately 50 µl dichloromethane were added to each reaction vessel. Fmoc-deprotection was performed by adding 3 times 0.8 ml of piperidine/DMA 1/4 (v/v) to each reaction vessel. Coupling-and deprotection times were increased as the synthesis proceeded, starting with 30 min and 3 times 3 min, respectively. Washings after coupling and Fmoc-deprotection steps were done with 6 times 1.2 ml DMA. After synthesis the peptidylresins were washed extensively with DMA, dichloromethane, dichloromethane/ether 1/1 (v/v) and ether respectively, and dried in the air (at least 2 h). Cleavage of the peptide and removal of the side chain protecting groups was performed by adding 6 times 200 µl TFA/water 19/1 (v/v) at 5 min intervals to each reaction vessel. 2.5 h after the first TFA addition the peptides were precipitated from the combined filtrates by the addition of 10 ml ether/pentane 1/1 (v/v) and cooling to -20°C. The peptides were isolated by centrifugation (-20°C, 2,500g, 10 min). After titration of the pellet with 10 ml ether/pentane 1/1 (v/v) and isolation by the same procedure, the peptides were dried at 40°C for 15 min. Each of the peptides was dissolved in 2 ml water (or 2 ml 10 vol% acetic acid), the solution frozen in liquid nitrogen for 3 min, and lyophilized while being centrifuged (1,300 rpm, 8-16 h). The peptides were stored at -20°C until use. The purity of the peptides was determined by analytical reversed phase HPLC using a water-acetonitrile gradient containing 0.1 % TFA, and proved to be at least 70% pure (UV 214 nm). The integrity of the peptides was determined by laser desorption time-of-flight mass spectrometry (MALDI-TOF MS) on a Lasermat mass spectrometer (Finnigan MAT, UK). About 5 pmol of the peptide in 0.5 µl water/acetonitrile containing 0.1% TFA was mixed with 0.5 µl of matrix solution (ACH, 10 mg/ml in acetonitrile/water 60/40 (v/v) containing 0.1% TFA) and applied to the instrument. Calibration was performed with peptides of known molecular mass (1422.7 or 1896.1), either as external or as internal references.

### Attachment of the carbohydrate moieties to the peptides

In the procedure described below α-D-mannopyranosylphenylisothiocyanate was used for the introduction of the mannose moieties in the peptides. The same procedure was followed for the synthesis of the peptides containing other glyco moieties.

To a solution of 2 mg of a synthetic peptide in 200 µl DMSO was added 2 mg of α-D-mannopyranosylphenylisothiocyanate (Cas 96345-79-8) per amino group in the peptide and 1 µl of NMM per amino group in the peptide, respectively.

The reactionmixture was stirred overnight, afterwhich the excess α-D-mannopyranosylphenylisothiocyanate was hydrolysed in 1 h by the addition of 25 µl Tris.HCL (pH 9.5, 1 M) per mg α-D-mannopyranosylphenylisothiocyanate used.

The mannosylated peptides were purified by gradient elution on a Jasco HPLC system, containing a Jasco PU-980 intelligent HPLC Pump, a Jasco LG-980-02 Ternary Gradient Unit, a Jasco UV-975 intelligent UV/VIS Detector, a Jasco AS-950 intelligent Sampler, controlled by a JCN Cobra Excel 486 PC using Borwin vs 1.20 for Windows (B&L Systems, Maarssen, The Netherlands) equipped with a RP-HPLC Vydac 218TP1010 column (1.0 x 25cm) using a flow of 5 ml/min and a linear gradient of water/acetonitrile/TFA 949/50/1 to water/acetonitrile/TFA 249/750/1 in 28 min. Detection was at 225 nm. The purified peptides were lyophilized in aliquots and stored at -20°C until use.

The purity of the mannosylated peptides was checked on a Waters LCM-1 system using Millenium Chromatography Manager (Waters) and equipped with a RP-HPLC Nucleosil 100-5C18AB column (0.4 x 25 cm)(Macherey-Nagel, Düren, Germany), using a flow of 1 ml/min and a linear gradient of water/acetonitrile/TFA 949/50/1 to water/acetonitrile/TFA 249/750/1 in 23 min. Detection was at 214 nm.

All mannosylated peptides were analysed by MALDI-TOF MS on a Lasermat mass spectrometer (Finnigan MAT, UK). About 5 pmol of the peptide in 0.5 µl water/acetonitrile containing 0.1% TFA was mixed on a sample slide with 0.5 µl of matrix solution (ACH, 10 mg/ml in acetonitrile/water 60/40 (v/v) containing 0.1% TFA). The sample was allowed to dry in the air for about 10 min and applied to the instrument. Calibration was performed with peptides of known molecular mass (1422.7 or 1896.1), either as external or as internal references.
The following peptides were synthesized:

| | |
|---|---|
| K1P1 | K-T-I-A-Y-D-E-E-A-R-R-G |
| K2P1 | K-K-T-I-A-Y-D-E-E-A-R-R-G |
| K3P1 | K-K-K-T-I-A-Y-D-E-E-A-R-R-G |
| K4P1 | K-K-K-K-T-I-A-Y-D-E-E-A-R-R-G |
| K5P1 | K-K-K-K-K-T-I-A-Y-D-E-E-A-R-R-G |
| K1P2 | K-P-S-V-Q-I-Q-V-Y-Q-G-E-R-E-I-A-S-H |
| K1P3 | K-L-Q-A-A-P-A-L-D-R-L |
| K1P4 | K-G-G-R-C-I-L-G-F-V-F-T-L |
| K1P1P4 | K-T-I-A-Y-D-E-E-A-R-R-G-G-R-G-I-L-G-F-V-F-T-L |
| M2K1P1 | M-K(M)-T-I-A-Y-D-E-E-A-R-R-G |
| M3K2P1 | M-K(M)-K(M)-T-I-A-Y-D-E-E-A-R-R-G |
| M4K3P1 | M-K(M)-K(M)-K(M)-T-I-A-Y-D-E-E-A-R-R-G |
| M5K4P1 | M-K(M)-K(M)-K(M)-K(M)-T-I-A-Y-D-E-E-A-R-R-G |
| M6K5P1 | M-K(M)-K(M)-K(M)-K(M)-K(M)-T-I-A-Y-D-E-E-A-R-R-G |
| M2K1P2 | M-K(M)-P-S-V-Q-I-Q-V-Y-Q-G-E-R-E-I-A-S-H |
| M2K1P3 | M-K(M)-L-Q-A-A-P-A-L-D-R-L |
| M2K1P4 | M-K(M)-G-G-R-G-I-L-G-F-V-F-T-L |
| M2K1P1P4 | M-K(M)-T-I-A-Y-D-E-E-A-R-R-G-G-R-G-I-L-G-F-V-F-T-L |

Mass spec data of the peptides:

| | Formula | MH⁺_{calc} | MH⁺ₘₑₐₛ |
|---|---|---|---|
| K1P1 | C₅₉H₉₇N₁₉O₂₁ | 1409.5 | 1409.1 |
| K2P1 | C₆₅H₁₀₉N₂₁O₂₂ | 1537.7 | 1536.9 |
| K3P1 | C₇₁H₁₂₁N₂₃O₂₃ | 1665.9 | 1666.2 |
| K4P1 | C₇₇H₁₃₃N₂₅O₂₄ | 1794.1 | 1793.6 |
| K5P1 | C₈₃H₁₄₅N₂₇O₂₅ | 1922.2 | 1922.2 |
| K1P2 | C₉₀H₁₄₅N₂₇O₂₉ | 2070.3 | 2068.5 |
| K1P3 | C₅₃H₉₄N₁₆O₁₅ | 1196.4 | 1196.6 |
| K1P4 | C₆₅H₁₀₅N₁₇O₁₅ | 1365.6 | n.d. |
| K1P1P4 | C₁₁₆H₁₈₅N₃₃O₃₃ | 2571.0 | 2570.0 |
| M2K1P1 | C₈₅H₁₂₇N₂₁O₃₃S₂ | 2036.2 | 2035.3 |
| M3K2P1 | C₁₀₄H₁₅₄N₂₄O₄₀S₃ | 2477.7 | 2476.5 |
| M4K3P1 | C₁₂₃H₁₈₁N₂₇O₄₇S₄ | 2919.2 | 2917.2 |
| M5K4P1 | C₁₄₂H₂₀₈N₃₀O₅₄S₅ | 3360.7 | 3360.9 |
| M6K5P1 | C₁₆₁H₂₃₅N₃₃O₆₁S₆ | 3802.2 | 3796.7 |
| M2K1P2 | C₁₁₆H₁₇₅N₂₉O₄₁S₂ | 2696.9 | 2697.3 |
| M2K1P3 | C₇₉H₁₂₄N₁₈O₂₇S₂ | 1823.0 | 1822.0 |
| M2K1P4 | C₉₁H₁₃₅N₁₉O₂₇S₂ | 1992.3 | 1992.1 |
| M2K1P1P4 | C₁₄₂H₂₁₅N₃₅O₄₅S₂ | 3197.6 | 3195.3 |

### Dendritic cell culture

Dendritic cells were cultured according to the protocol described by Sallusto and Lanzavecchia. Routinely the cells were cultured for 7 days. All dendritic cell cultures were checked for the expression of typical dendritic cell surface markers by fluorescence activated cell sorter analysis on day 7. The cell surface markers analysed included CD1a, HLA-class I and class II, CD4 and the mannose receptor. All cultures were found to be more than 95% positive for these markers. Subsequently the cells were frozen in a mixture of RPMI 1640 (Gibco) supplemented with 20% serum and 10% DMSO and stored in liquid nitrogen until usage.

### T CELL CLONES

The T cell clones have been described in detail elsewhere (Anderson et al, Geluk et al, Oftung et al). Clone RP15.1.1 is specific for the peptide corresponding to amino acid 3-13 of Mycobacterium leprae HSP65. This peptide is designated P1. The response to the peptide is HLA-DR3 restricted. The T cell clone R3F7 is specific for the peptide corresponding to amino acid 413-424 of the Mycobacterium leprae HSP70. This peptide is designated P2. The response to the peptide is HLA-DR2 restricted. The T cell clone R2F10 is specific for the peptide corresponding to amino acid 418-427 of the Mycobacterium leprae HSP60. This peptide is designated P3. The response to the peptide is HLA-DR2 restricted.
The T cell clones were expanded by specific stimulation with the appropiate peptide in the presence of autologous irradiated (3000 RAD) peripheral blood mononuclear cells as antigen presenting cells. Subsequently the cells were frozen in a mixture of RPMI 1640 supplemented with 20% serum and 10% DMSO and stored in liquid nitrogen until usage.

### Proliferation assay

For the measurement of proliferation 10.000 cloned T cells were mixed with 10.000 irradiated cultured dendritic cells. To this mannosylated or non-mannosylated peptide was added in a previously determined concentration range. The total volume of the culture was 200 µl. All cultures were carried out in triplicates. Culture was at 37°C in a 5% CO₂ incubator for 48 hours. Next 1 µCi [³H]-thymidine was added and the culture continued for another 16 hours. Subsequently the cultures were harvested and the [³H]-thymidine incorporation was measured using a beta-plate counter (Pharmacia) using standard protocols.

### RESULTS

We have investigated if the in vitro mannosylation of immunogenic peptide enhances their immunogenicity. For this purpose synthetic variants of three known antigenic peptides were synthetized with the addition of an N-terminal lysine residue. The peptides used were (single amino acid code); K-T-I-A-Y-D-E-E-A-R-R-G (designated peptide KP1), K-P-S-V-Q-I-Q-V-Y-Q-G-E-R-E-I-A-S-H (designated peptide KP2) and K-L-Q-A-A-P-A-L-D-R-L (designated peptide KP3). The P1 peptide binds specifically to HLA-DR3 and is recognized by the human T cell clone RP15.1.1. The P2 and P3 peptide are recognized by the human T cell clones R3F7 and R2F10 respectively when bound to HLA-DR2. A portion of these peptides was bis-mannosylated by the covalent attachment of mannose to both available primary amines on the N-terminal lysine residue. The peptides are designated M-K(M)-T-I-A-Y-D-E-E-A-R-R-G (M2KP1), M-K(M)-P-S-V-Q-I-Q-V-Y-Q-G-E-R-E-I-A-S-H (M2KP2) and M-K(M)-L-Q-A-A-P-A-L-D-R-L (M2KP3) where M stands for an covalently linked mannose moiety. Both the mannosylated and non-mannosylated forms of these peptides were purified by preparative reversed phase HPLC. The correct mannosylation was checked by mass spectral analysis of the purified product. The exact protocol used is described herein.

Human monocyte-derived dendritic cells were cultured according to a standard protocol described above. The monocytes were isolated from the peripheral blood of an HLA-DR3 (DR17, DRbetal*0301) positive and an HLA-DR2 positive healthy individual. The dendritic cells were co-cultured with the appropiate peptide specific T cells clones in the presence or absence of a concentration range of both the mannosylated and non-mannosylated versions of the peptides. Specific proliferation was measured by the incorporation of [³H]-thymidine after 48 hours of culture. A similar experiment was performed using peripheral blood mononuclear cells as antigen presenting cells instead of dendritic cells. The exact protocol used is described herein.

In those experiments where dendritic cells were used as antigen presenting cells and the T cell clones R2F10 and RP15.1.1 as responding cells it was observed that for the induction of half maximal proliferation approximately 3,000-to 10,000-fold less of the mannosylated peptide was required compared to the non-mannosylated analog. In those experiments where dendritic cells were used as antigen presenting cells and the T cell clone R3F7 as responding cell it was observed that for the induction of half maximal proliferation approximately 100- to 300-fold less of the mannosylated peptide was required compared to the non-mannosylated analog.

In those experiments where peripheral blood mononuclear cells were used as antigen presenting cells and the T cell clones R2F10 and RP151.1 as responding cells it was observed that for the induction of half maximal proliferation approximately 1,000- to 3,000-fold less of the mannosylated peptide was required compared to the non-mannosylated analog. In those experiments where peripheral blood mononuclear cells were used as antigen presenting cells and the T cell clone R3F7 as responding cell it was observed that for the induction of half maximal proliferation approximately 3- to 10-fold less of the mannosylated peptide was required compared to the non-mannosylated analog.

It is of importance to note that the clone R3F7 requires at least 10-fold less non-mannosylated peptide for the induction of half-maximal proliferation compared to the clones R2F10 and RP15.1.1 and that the diminished effect of mannosylation in the case of the clone R3F7 may relate to the higher sensitivity of this clone.

In all experiments in which dendritic cells were used as antigen presenting cells, clone RP15.1.1 as responder cell and M2KP1 peptide, half maximal proliferation was observed at a peptide concentration of approximately 1 femtomol/ml. For the other two clones this was approximately 10 to 30 femtomol/ml. In the culture system used this corresponds to the availability of approximately 10,000 to 300,000 bis-mannosylated peptides for each dendritic cell. Since the number of mannose binding sites on cultured human dendritic cells is estimated to be 1.7 x 10⁶ (Avrameas, A., et al. (1996)) this indicates that an absolute shortage of the mannosylated peptide resulting in suboptimal occupance of the mannose receptor on the dendritic cells directly correlates with diminished antigenic stimulation of the responding peptide specific T cells.

Next we tested the influence of multiple mannose residues on the P1 peptide on presentation by dendritic cells. For this purpose the P1 peptide was synthetized with the addition of 1, 2, 3, 4 and 5 lysine residues at the N-terminus (KP1, K2P1, K3P1, K4P1 and K5P1). These peptides were subsequently mannosylated and purified (M2KP1, M3K2P1, M4K3P1, M5K4P1 and M6K5P1) and tested for their capacity to induce proliferation of the T cell clone RP15.1.1 in the presence of DR3 positive cultured dendritic cells. Virtually identical dose response-curves were obtained with all these peptides, inducing half-maximal proliferation at a concentration of approximately 1 femtomol/ml. Thus, under these conditions the addition of more than two mannose residues to the peptide appears not relevant for uptake and presentation by dendritic cells to T cells.

Finally we tested if a short exposure of dendritic cells to a mannosylated peptide was sufficient for the induction of proliferation of the T cell clone RP15.1.1. Dendritic cells were incubated at 37°C with either P1 or M2KP1 peptide and either washed directly with excess ice cold medium (0 minute incubation time point) or after a 3 and 10 minute incubation period. Subsequently the washed dendritic cells were co-cultured with clone RP15.1.1 and the proliferation measured after 48 hours. At both the 3 and 10 minute time point both the mannosylated (M2KP1) and the non-mannosylated peptide (P1) are able to induce strong proliferation of the T cell clone. At the 0 min time point, however, only the mannosylated peptide (M2KP1) induces strong proliferation. Thus, binding of mannosylated peptide to the receptor is fast and only a short exposure of the dendritic cell to a mannosylated ligand is required for the internalisation and subsequent presentation of an immunogenic peptide.

### LEGEND

Table 1 gives a list of peptides which are very suitable to be used according to the present invention.
This list is by no means exhaustive or limiting.

### ABBREVIATIONS

- ACH: α-cyano-4-hydroxycinnamic acid
- DC: Dendritic cell
- DMA: N,N-dimethylacetamide
- DMSO: Dimethylsulfoxide
- Fmoc: 9-fluorenylmethoxycarbonyl
- HSP: Heat shock protein
- NMM: N-methylmorpholine
- NMP: N-methylpyrrolidone
- PyBop: Benzotriazole-1-yl-oxy-tris-pyrrolidino-phosphonium hexafluorophosphate
- TFA: Trifluoroacetic acid

### REFERENCES

Coste, J., Dufour, M.-N., Nguyen, D. and Castro, B. (1990) BOP and congenes: present status and new developments. In: J.E. Rivier and G.R. Marshall (Eds.) Peptides: Chemistry, Structure and Biology, Escom, Leiden, p. 885.

Fields, G.B. and Noble, R.L. (1990) Solid phase peptide synthesis utilizing 9-fluorenylmethoxycarbonyl amino acids. Int. J. Peptide Protein Res. 35, 161.

Gausepohl, H., Kraft, M., Boulin, C. and Frank, R.W. (1990^{a}) A multiple reaction system for automated simultaneous peptide synthesis. In: E. Giralt and D. Andreu (Eds.) Peptides 1990, Escom, Leiden, p. 206.

Gausepohl, H., Kraft, M., Boulin, Ch. and Frank, R.W. (1990^{b}) Automated multiple peptide synthesis with BOP activation. In: J.E. Rivier and G.R. Marshall (Eds.), Peptides: Chemistry, Structure and Biology, Escom, Leiden, p.1003.

Rapp, W., Zhang, L. and Bayer, E. (1990) Continuous flow peptide synthesis on PSPOE-graft copolymers. In: R. Epton (Ed.) Innovation and Perspectives in Solid Phase Peptide Synthesis, SPCC (UK) Ltd, Birmingham, p. 205.

Sheppard, R.C. and Williams, B.J. (1982) Acid-labile resin linkage agents for use in solid phase peptide synthesis. Int. J. Peptide Protein Res. 20, 451.

Anderson, D.C., Van Schooten, W.C.A., Janson, A., Barry, M.E., and De Vries, R.R.P. (1990). Molecular mapping of interactions between a Mycobacterium leprae-specific T cell epitope, the restricting HLA-DR2 molecule, and two specific T cell receptors. J. Immunol. *144*, 2459-2464

Geluk, A., Van Meijgaarden, K.E., Janson, A.A.M., Drijfhout, J.W., Meloen, R.H., De Vries, R.R.P., and Ottenhoff, T.H.M. (1992). Functional analysis of DR17(3)-restricted mycobacterial T cell epitopes reveals DR17 binding motif and enables the design of allele specific competitor peptides. J. Immunol *149*, 2864-2871

Offung, F., Geluk, A., Lundin, K.E.A., Meloen, R.H., Thole, J.E.R., Mustafa, A.S., and Ottenhoff, T.H.M. (1994). Mapping of multiple HLA class II-restricted T-cell epitopes of the mycobacterial 70-kilodalton heat shock protein. Infection and Immunity *62*, 5411-5418

Sallusto, F. and Lanzavecchia, A. (1994). Efficient presentation of soluble antigen by cultured human dendretic cells is maintained by granulocyte/macrophage colony-stimulating factor plus interleukin 4 and downregulated by tumor necrosis factor α. J. Exp.Med. *179*, 1109-1116

Avrameas, A., McIlroy, D., Hosmalin, A., Autran, B., Debré, P., Monsigny, M., Roche, A.C., and Midoux, P. (1996). Expression of a mannose/fucose membrane lectin on human dendritic cells. Eur. J. Immunol. 26, 394-400

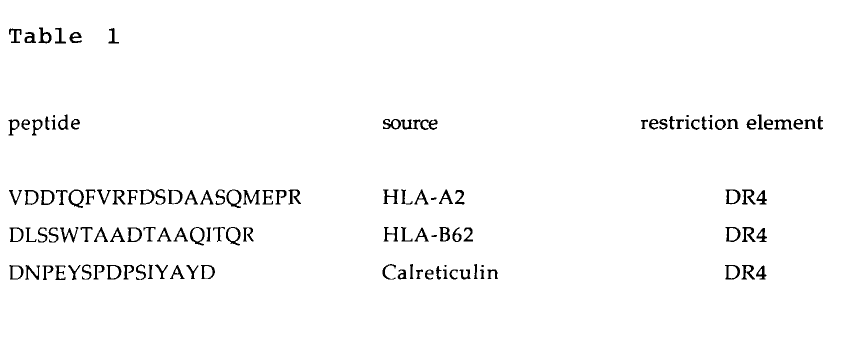

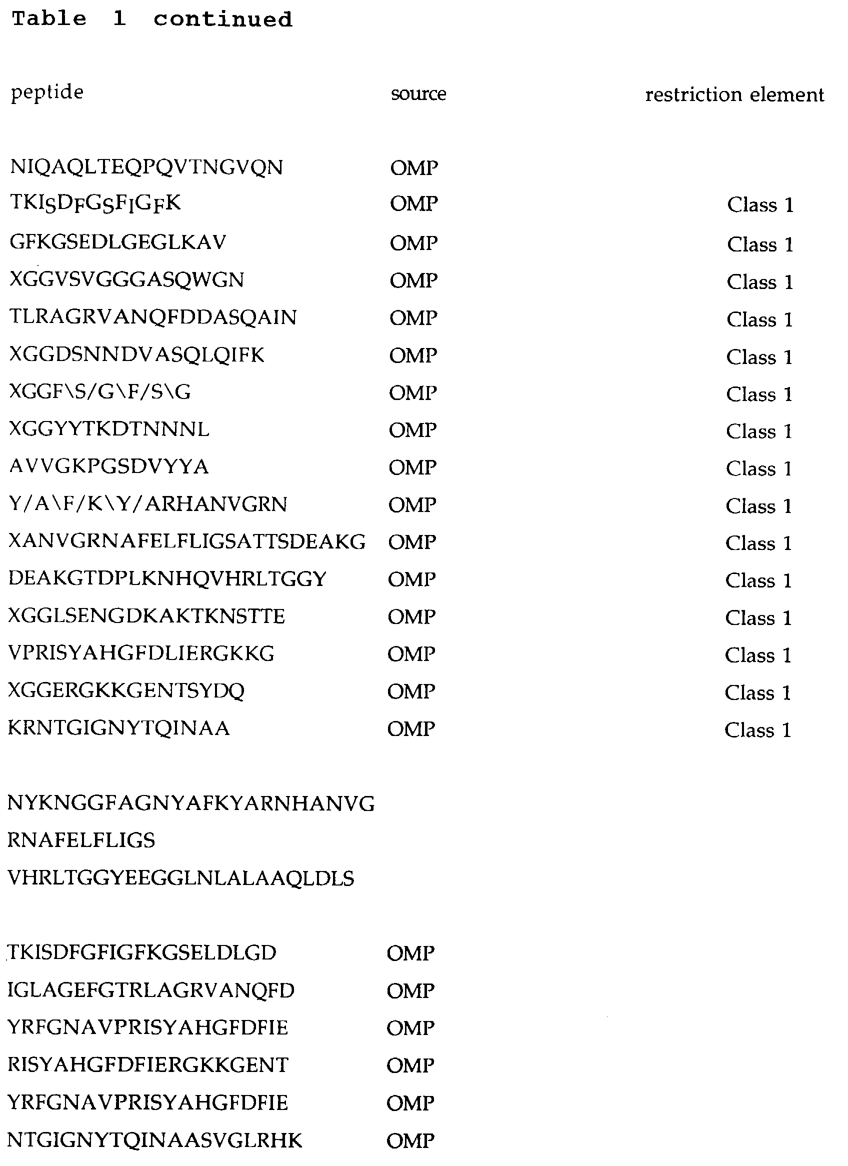

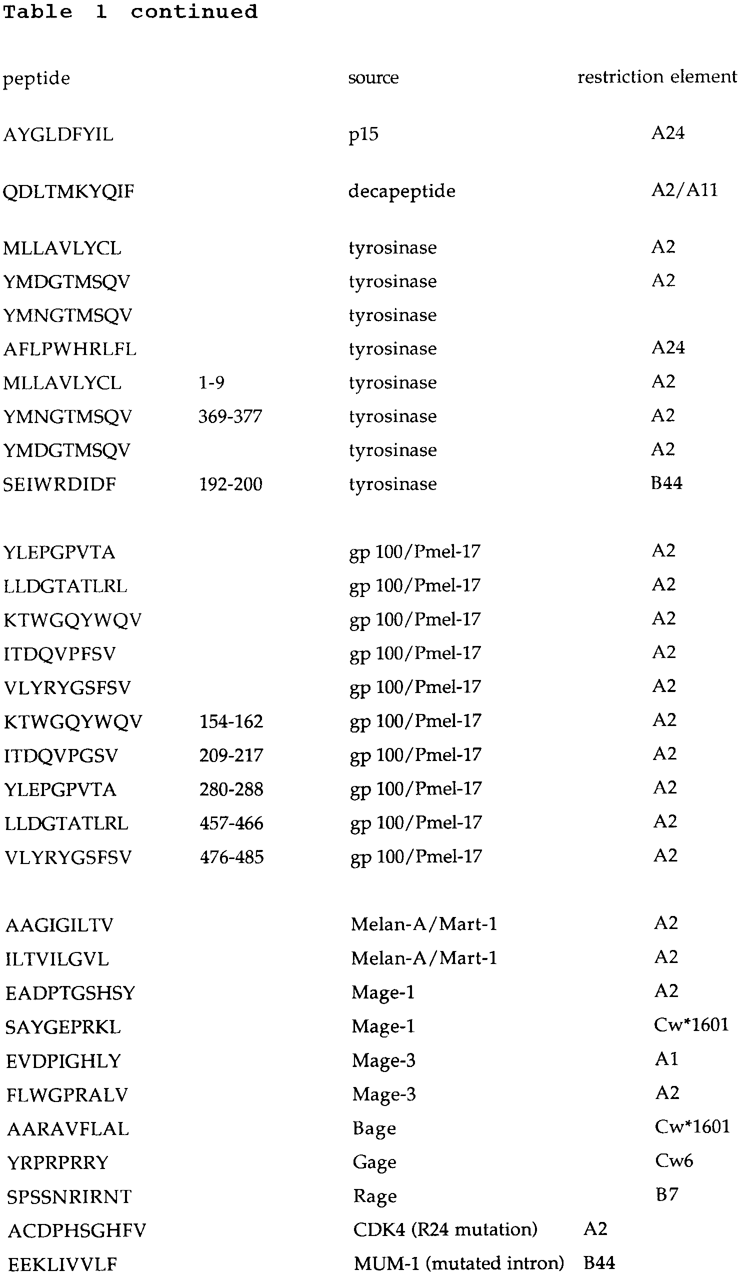

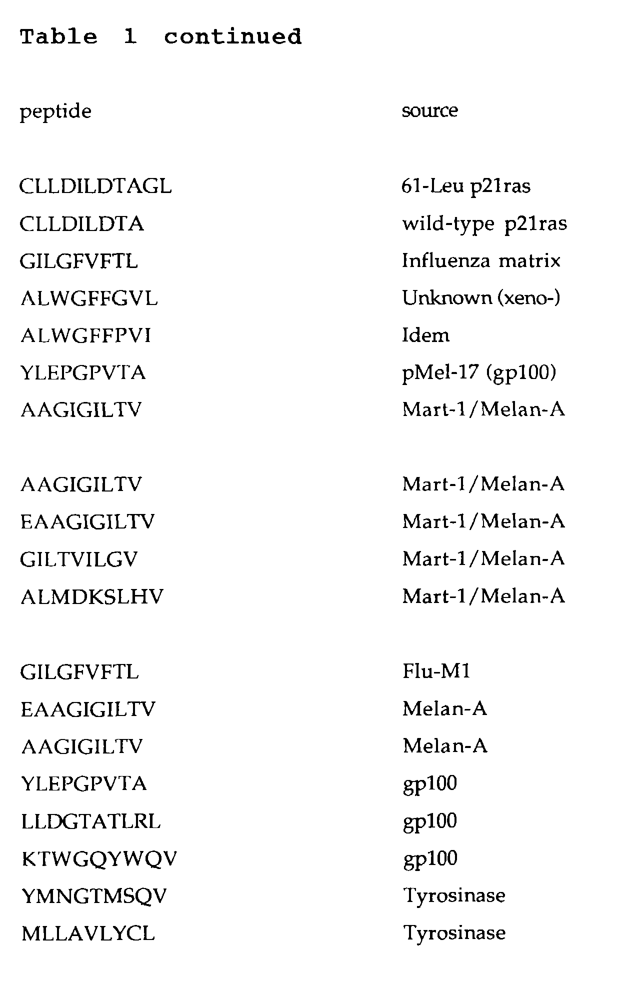

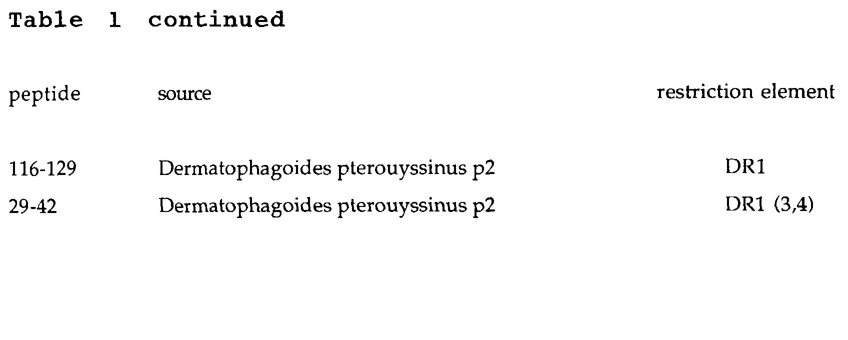

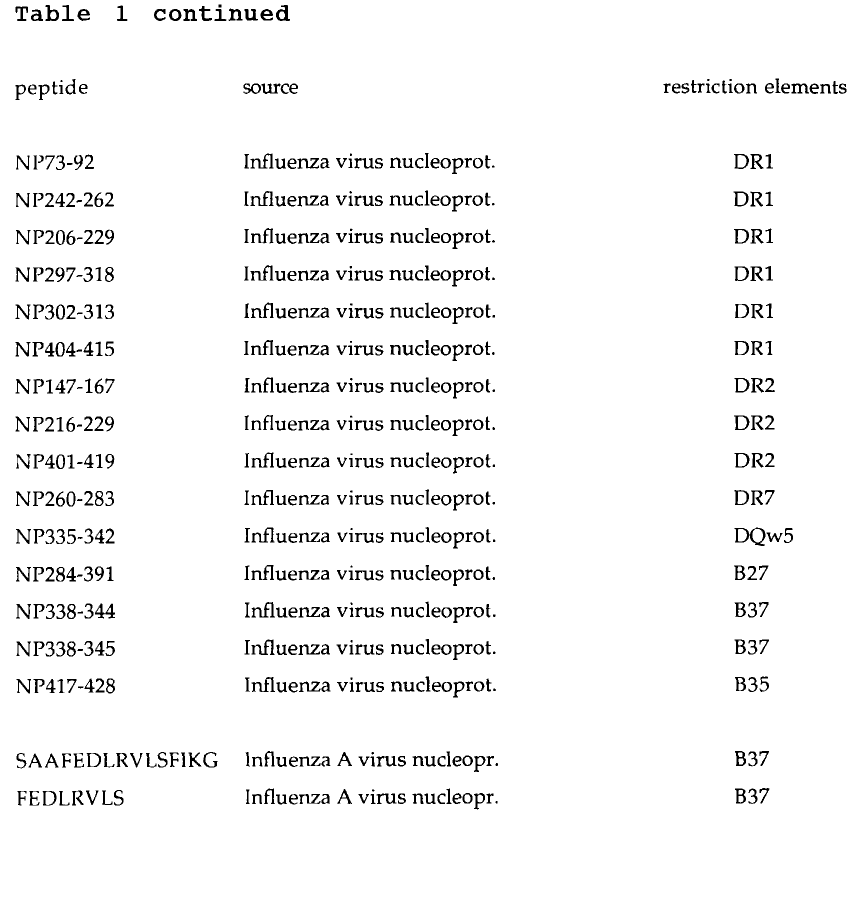

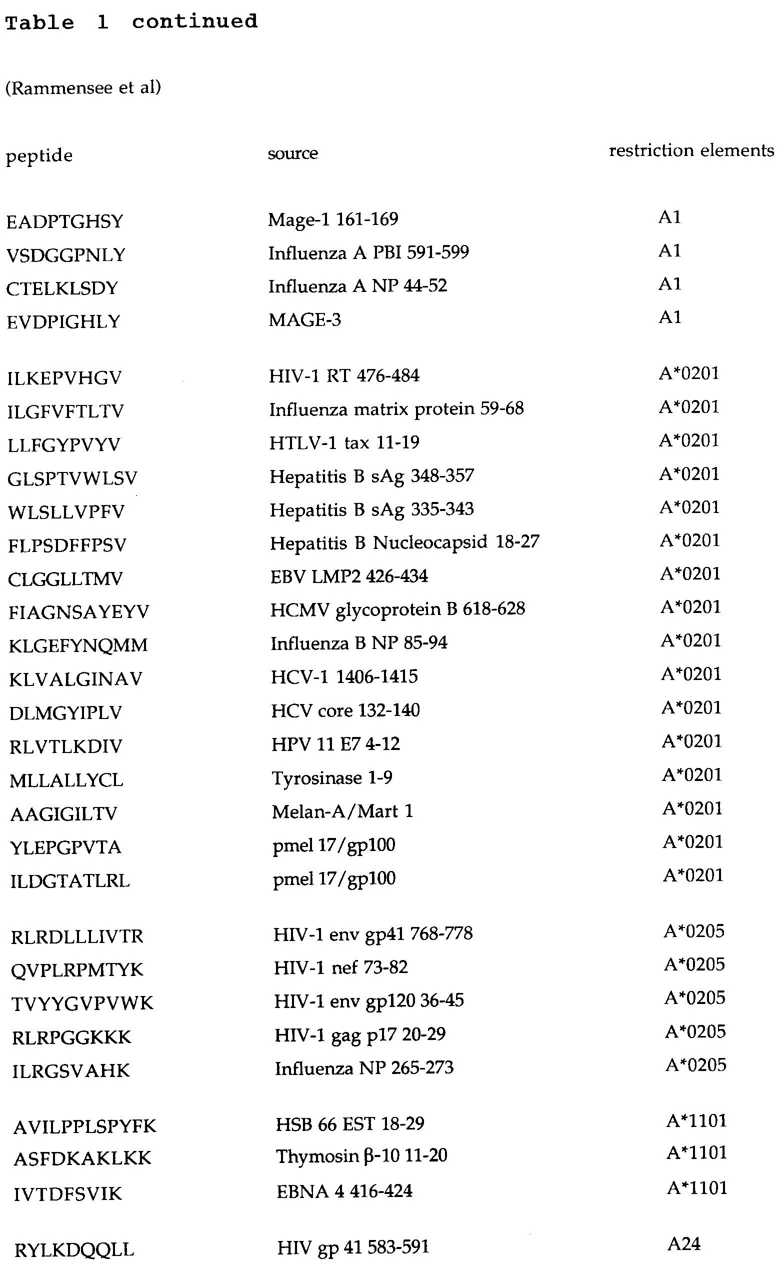

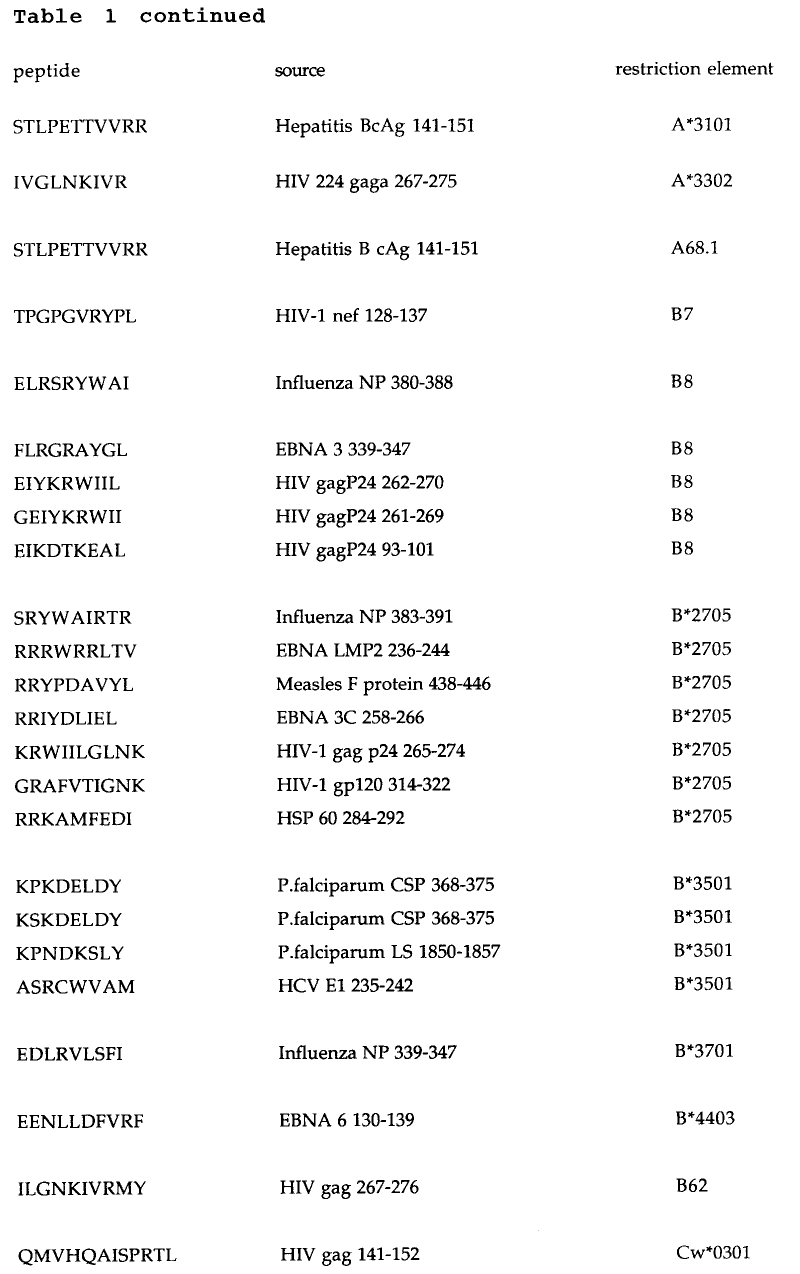

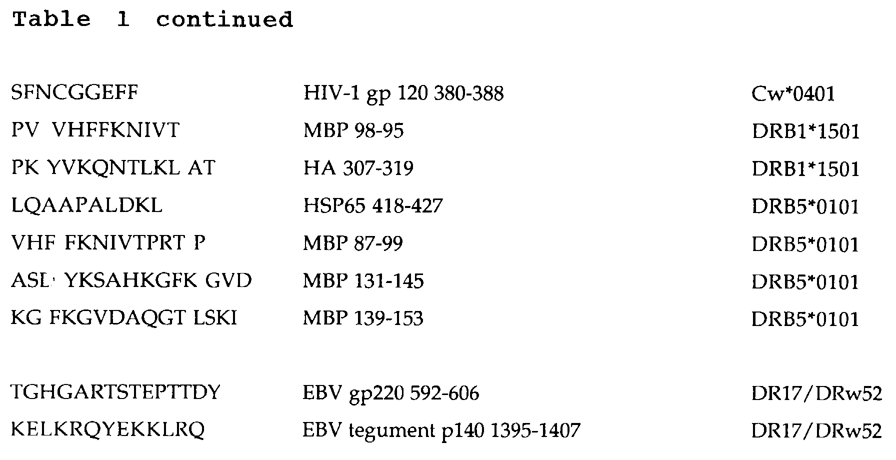

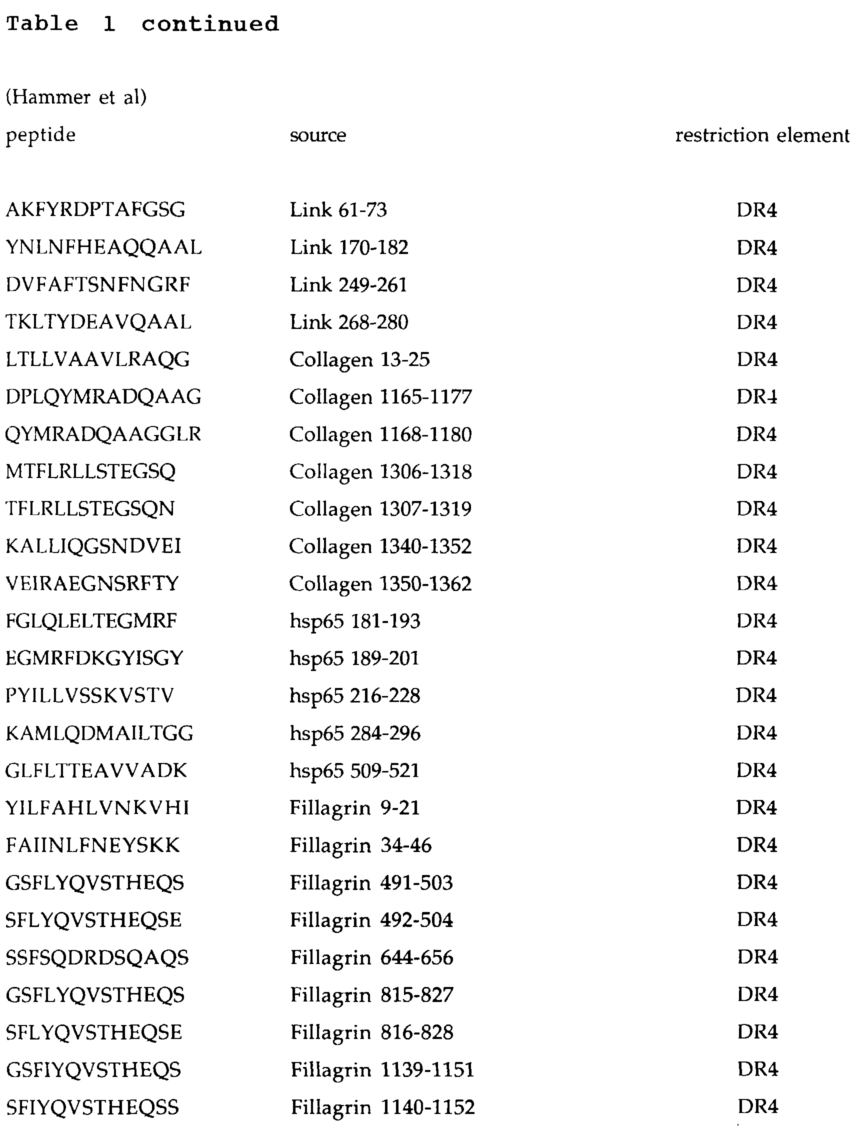

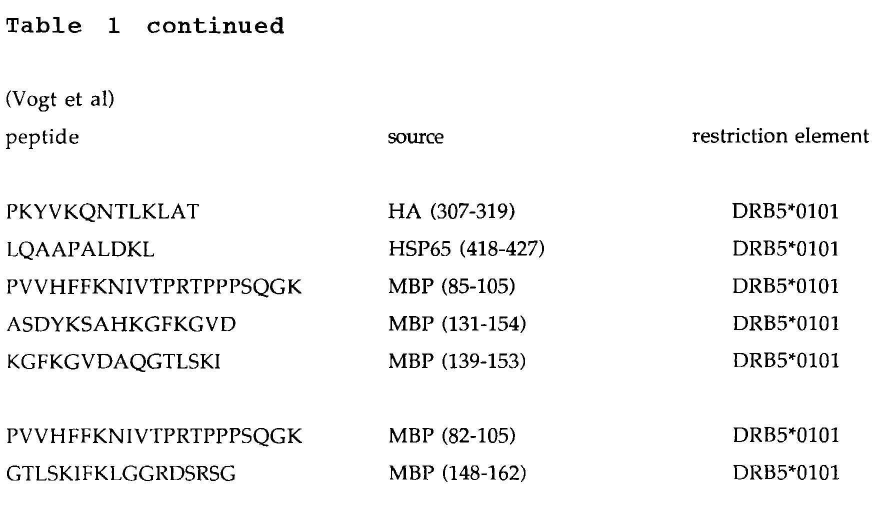

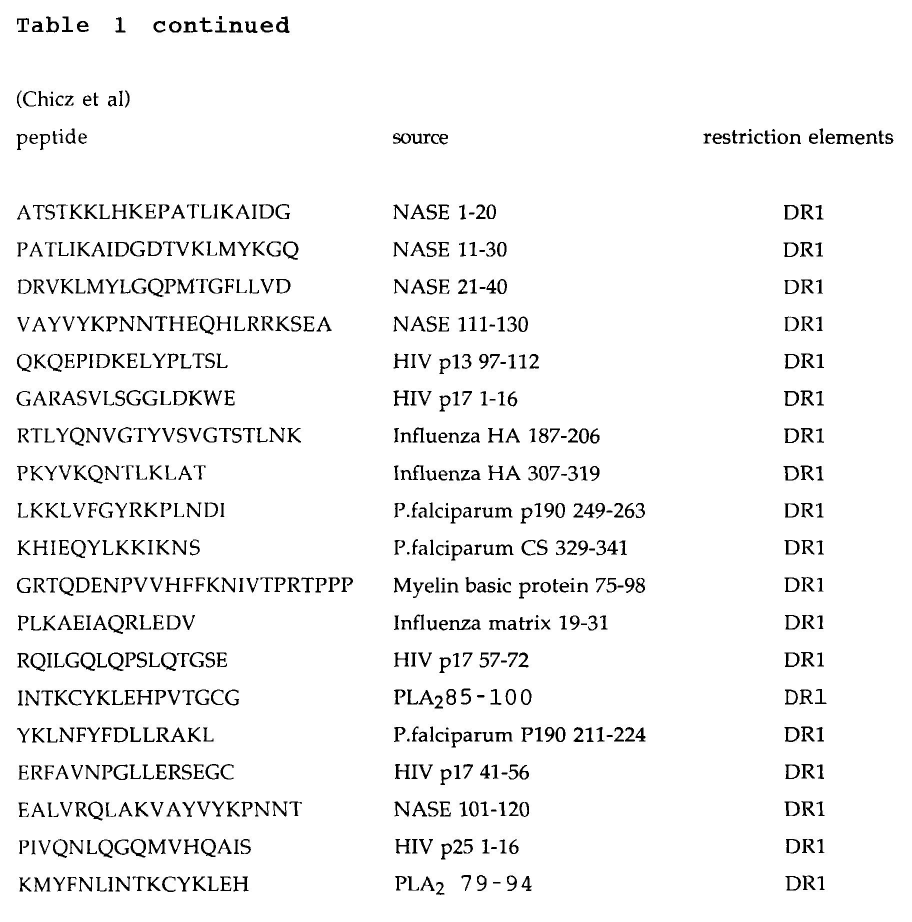

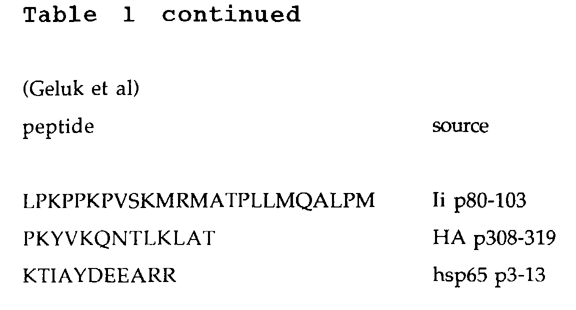

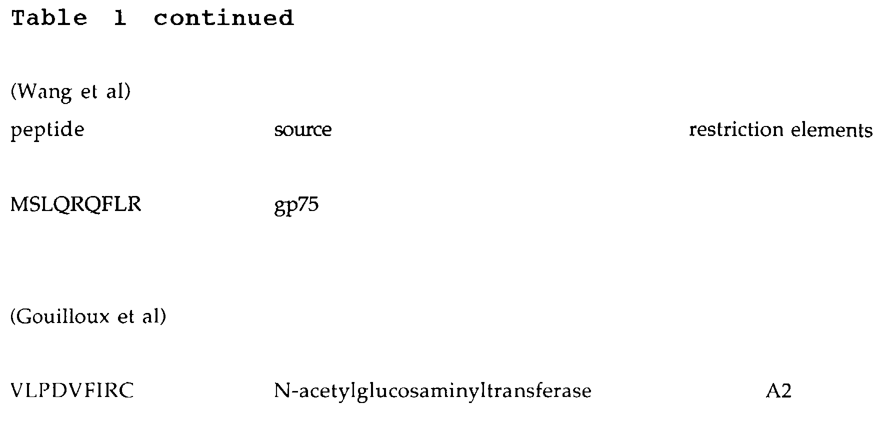

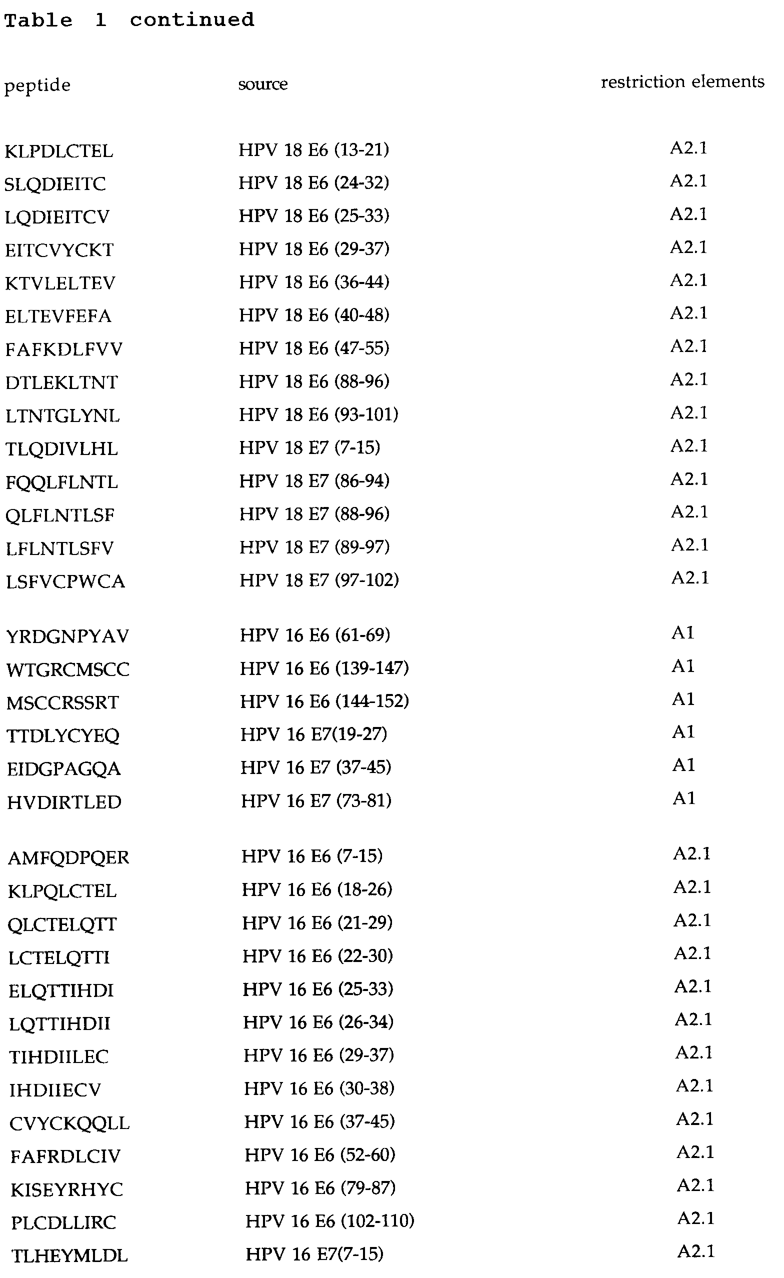

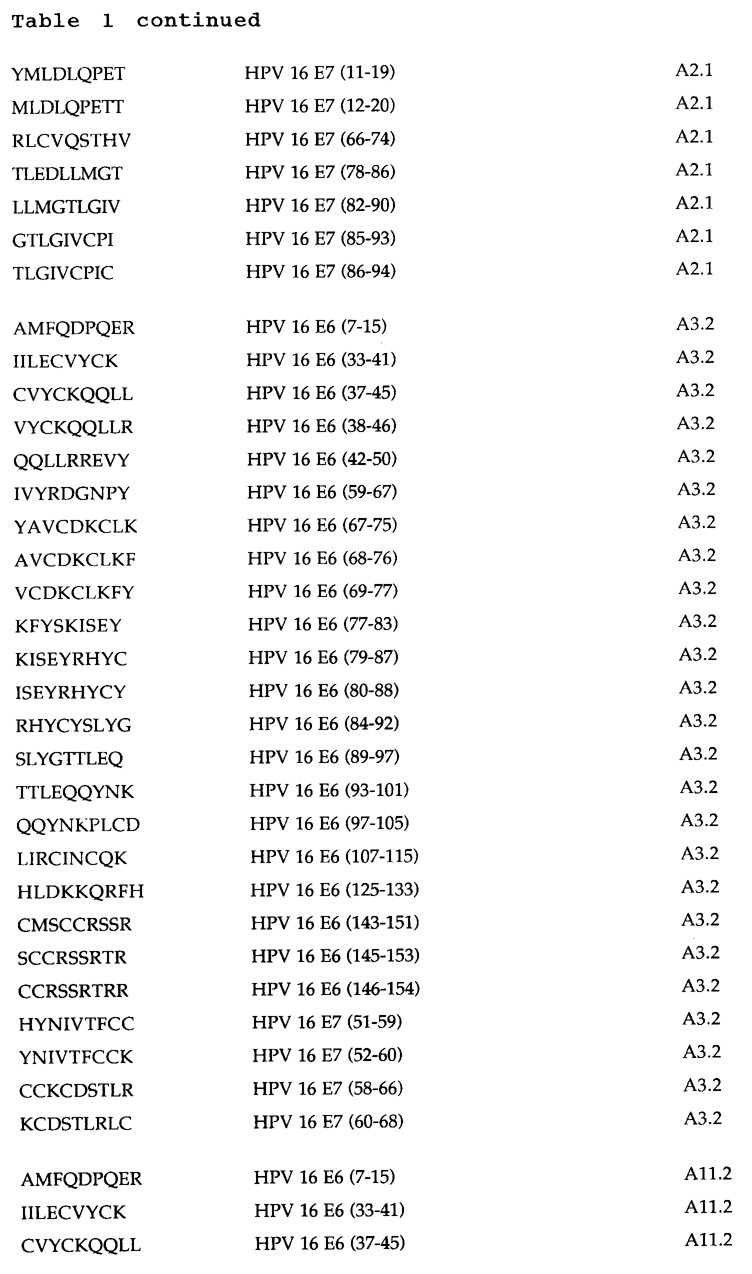

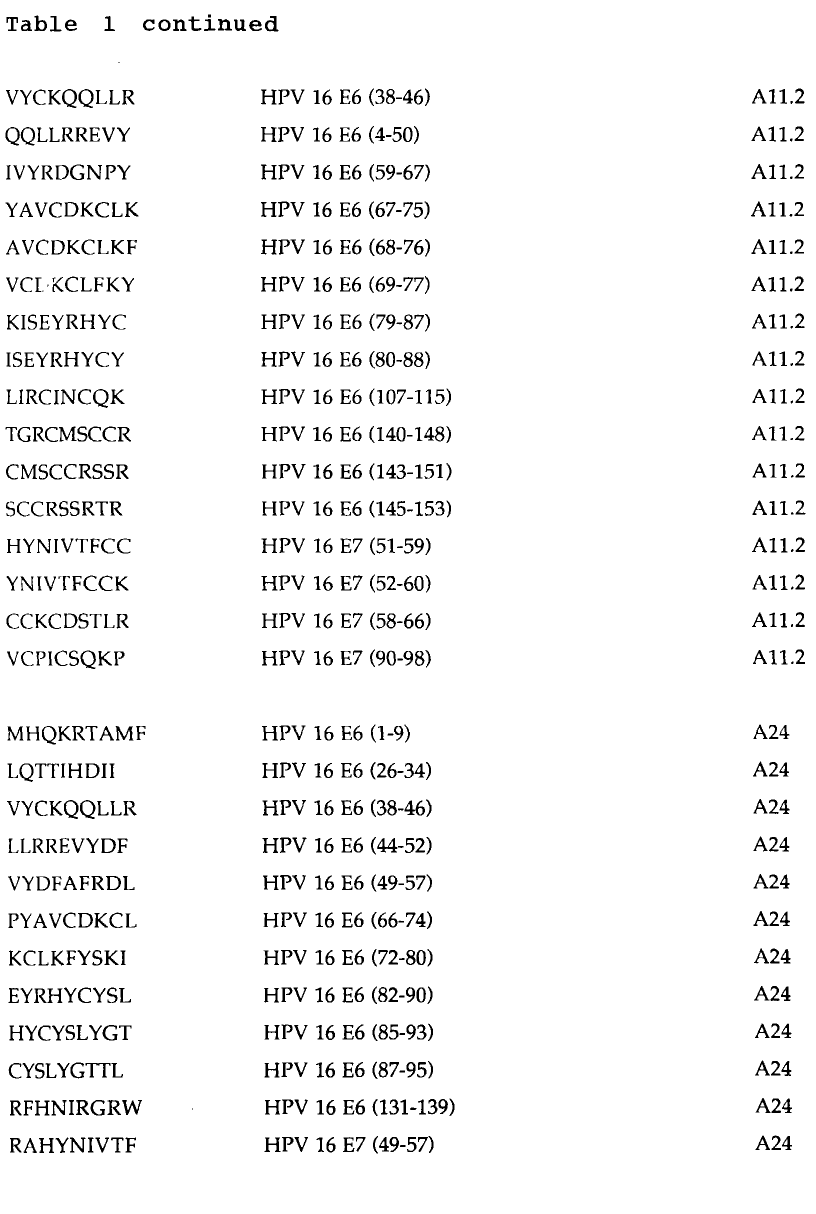

## Claims

1. A method for enhancing uptake of a proteinaceous antigen or a peptide derived therefrom by antigen presenting cells having a mannose or similar receptor, whereby the antigen is provided with at least one and preferably two mannose groups or a functional equivalent thereof.

2. A method for enhancing antigen presentation by an antigen presenting cell comprising contacting said cell with a soluble antigen which has been provided with at least one and preferably two mannose groups or a functional equivalent thereof and allowing the cell to internalize and process said mannosylated antigen.

3. A method for producing a cell presenting a peptide antigen, whereby said peptide antigen is provided with at least one and preferably two mannose groups or a functional equivalent thereof, comprising contacting said cell with a soluble antigen which has been provided with a mannose group or a functional equivalent thereof and allowing the cell to internalize and process said mannosylated antigen.

4. A method according to claims 1-3 whereby the antigen presenting cell is a cell which presents peptides in the context of MHC-class II and/or MHC-class I.

5. A method according to claims 1-4 whereby the antigen presenting cell is a dendritic cell.

6. An antigen presenting cell obtainable by a method according to claim 3.

7. A cell according to claim 6 which is a dendritic cell.

8. A method for improving the T cell response to a peptide antigen comprising contacting said T cell with a cell according to claim 6 or 7.

9. A vaccine preparation for the treatment or prophylaxis of infection by a pathogen comprising at least one proteinaceous antigen derived from said pathogen, which proteinaceous antigen is provided with at least one and preferably two mannose groups or a functional equivalent thereof.

10. A vaccine preparation for the treatment or prophylaxis of infection by a pathogen comprising at least one peptide derived from said pathogen which peptide is provided with at least one and preferably two mannose groups or a functional equivalent thereof.

11. A peptide provided with at least one and preferably two mannose groups or a functional equivalent thereof for use in the preparation of a vaccine.

12. A proteinaceous antigen provided with at least one and preferably two mannose groups or a functional equivalent thereof for use in the preparation of a vaccine.
